# EUROPÄISCHE PATENTSCHRIFT

(11) **EP 2 233 814 B1**
(45) Veröffentlichungstag und Bekanntmachung des Hinweises auf die Patenterteilung: **04.05.2016**
(21) Anmeldenummer: 10001975.1
(22) Anmeldetag: 26.02.2010
(51) Int. Cl.: F16L 33/22

(54) **Vorrichtung zum lösbaren Verbinden mit einem Ende einer röhrenartigen Leitung, insbesondere Schlauch-Schnellverbinder**
Device for removably connecting to an end of a tubular conduit, in particular quick hose connector
Dispositif de connexion amovible doté d'une extrémité d'une conduite de type tubulaire, notamment connecteur rapide de tuyau

(30) Priorität: 23.03.2009 DE 102009015640
(43) Veröffentlichungstag der Anmeldung: 29.09.2010
(73) Patentinhaber: Eisele Pneumatics GmbH & Co. KG, 71332 Waiblingen (DE)
(72) Erfinder: Müller, Bernhard, 71397 Leutenbach (DE)
(74) Vertreter: Crazzolara, Helmut

(56) Entgegenhaltungen:
- DE-A1- 10 233 862
- US-A- 4 257 629
- US-A1- 2006 202 478

## Beschreibung

Die Erfindung betrifft eine Vorrichtung zum lösbaren Verbinden mit einem Ende einer röhrenartigen Leitung, insbesondere einen Schlauch-Schnellverbinder, mit den Merkmalen im Oberbegriff von Anspruch 1. Aus der DE 100 26 464 A1 ist eine Vorrichtung bekannt, die einen Grundkörper aufweist und ein Klemmelement, mittels dem eine Leitung an der Vorrichtung lösbar fixierbar ist. Das Fixieren erfolgt dabei mittels einer von dem Klemmelement ausgebildeten Klemmkante, die beim verbindenden Betätigen der Vorrichtung in die Außenseite der Leitung eingreift und dadurch die Leitung klemmend fixiert. Um eine totraumfreie Verbindung bereitzustellen, weist der Grundkörper eine Anlagefläche auf, an welche das stirnseitige Ende der Leitung in dichtende Anlage bringbar ist. Das Leitungsende ist vor dem klemmenden Betätigen der Vorrichtung ausreichend weit in die Aufnahmeöffnung des Grundkörpers einzuschieben. Wenn dies gewährleistet ist, kann mit der bekannten Vorrichtung nicht nur eine dauerhaft dichte, sondern auch eine im Wesentlichen totraumfreie Verbindung der Leitung bereitgestellt werden.

Die US 4 257 629 beschreibt eine Vorrichtung zum Verbinden mit einem Ende einer röhrenartigen Leitung, insbesondere Schlauch-Schnellverbinder, mit einem eine Längsachse aufweisenden Grundkörper und mit einem Klemmelement, mittels dem die Leitung an der Vorrichtung lösbar fixierbar ist, wobei der Grundkörper einen Nippel aufweist, auf den die Leitung aufschiebbar ist, wobei der Nippel auf seiner Außenfläche einen von der Zylinderform abweichenden Abschnitt aufweist, an dem die Leitung mittels des Klemmelements in dichtender Anlage fixierbar ist, wobei das Klemmelement ein Klemmmittel aufweist.

Der Erfindung liegt die Aufgabe zugrunde, eine gattungsgemäße Vorrichtung mit weiter verbesserten Gebrauchseigenschaften bereitzustellen. In einer Ausführungsart soll die Anwendung der erfindungsgemäßen Vorrichtung vereinfacht sein und dennoch dauerhaft eine dichte Verbindung gewährleistet sein.

Diese Aufgabe wird durch eine Vorrichtung mit den Merkmalen von Anspruch 1 gelöst. Vorteilhafte Ausführungsformen der Erfindung gehen aus den Unteransprüchen hervor.

Gemäß dem Kennzeichen von Anspruch 1 ist vorgesehen, dass das Klemmmittel in Richtung der Längsachse über den von der Zylinderform abweichenden Abschnitt des Nippels und in Richtung der Längsachse über das der Leitung zugewandte Ende des Nippels hinausragt, wodurch die Leitung in dichte Anlage an das stirnseitige Ende des Nippels bringbar ist, jedenfalls in dichte Anlage an einen Übergang von dem von der Zylinderform abweichenden Abschnitt, insbesondere der Kegelfläche, zu dem stirnseitigen Ende des Nippels, und dadurch die Totraumfreiheit der Verbindung weiter verbessert ist.

Es ist vorgesehen, dass der Grundkörper einen Nippel aufweist, auf den die Leitung aufschiebbar ist, und dass der Nippel einen Abschnitt aufweist, der auf seiner Außenfläche eine von der Zylinderform abweichende Form aufweist, und an dem die Leitung mittels des Klemmelements in dichtender Anlage fixierbar ist. Durch das Fixieren der Leitung im Bereich dieses Abschnitts ist selbst dann eine dichte Verbindung der Leitung mit der Vorrichtung gewährleistet, wenn die Leitung nur teilweise auf den Nippel aufgeschoben ist. Das Klemmen erfolgt von radial außen mittels des Klemmelements, wobei die Leitung radial innen durch den Stutzen abgestützt ist. Vorzugsweise kommt es beim Aufschieben der Leitung auf den Nippel noch nicht zu der dichten Anlage der Leitung an dem von der Zylinderform abweichenden Abschnitt, sondern erst durch das Betätigen des Klemmelements ist die Leitung in dichte Anlage an den Nippel bringbar. Dadurch kann die Leitung mit relativ geringem Kraftaufwand in den Grundkörper eingeführt und insbesondere auf den Nippel aufgesteckt werden, ohne dass es zu einer relevanten Pressung an der Außenumfangsfläche des Nippels kommt.

In einer Ausführungsart weist der von der Zylinderform abweichende Abschnitt eine sich in Richtung auf das der Leitung zugewandte Ende des Nippels verjüngende Kegelform auf. Alternativ oder ergänzend kann der Nippel auch eine sonstige geeignete Form, beispielsweise eine gekrümmte, insbesondere teilsphärische Form aufweisen. Durch die Verjüngung in Richtung auf das freie Ende des Nippels ist das Aufscheiben der Leitung auf den Nippel vereinfacht und/oder eine Zentrierung der Leitung in Bezug auf die Vorrichtung gewährleistet.

In einer Ausführungsart weist der Nippel den von der Zylinderform abweichenden Abschnitt an oder nahe seinem der Leitung zugewandten Ende auf. Die Leitung kann sich beim Klemmen an das freie Ende des Nippels derart anlegen, dass die Nahtstelle zwischen Leitung und Nippel im Wesentlichen totraumfrei ist. Dadurch ist nicht nur eine dichte, sondern auch eine totraumfreie Verbindung von Leitung und Vorrichtung gewährleistet.

In einer Ausführungsart weist das Klemmelement für den klemmenden Kontakt mit der Leitung das Klemmmittel auf, das im Bereich des von der Zylinderform abweichenden Abschnitts des Nippels in Anlage an die Leitung bringbar ist. Insbesondere kann das Klemmmittel von radial außen in Anlage an die Leitung gebracht werden, und der Nippel bringt von radial innen eine Gegenkraft auf die Leitung auf, durch welche die Dichtwirkung weiter verbessert ist.

In einer Ausführungsart weist das Klemmelement das Klemmmittel auf, das vorzugsweise einstückig eine Klemmfläche ausbildet, wobei die Klemmfläche ausreichend groß bemessen ist, um beim Klemmen die auf die Leitung wirkende Flächenpressung, die als Kraft pro Fläche auch auf die Klemmfläche des Klemmmittels wirkt, auf einen vorgebbaren Wert zu begrenzen. Die Flächenpressung kann dabei u. a. durch die Wahl der Größe und/oder Form der Klemmfläche ausreichend groß bemessen sein, so dass die Dichtwirkung gewährleistet ist. Andererseits kann die Flächenpressung u. a. durch die Wahl der Größe und/oder Form der Klemmfläche aber auch dahingehend beschränkt sein, dass ein Fließen des Werkstoffes der Leitung verhindert oder jedenfalls so weit begrenzt ist, dass dadurch die Dichtwirkung auch langfristig nicht negativ beeinträchtigt ist. Die Größe der Klemmfläche ist dabei abhängig von dem Werkstoff der Leitung und von der radialen Auslenkung des Klemmmittels zu wählen.

In einer Ausführungsart weist das Klemmelement das Klemmmittel auf, das den von der Zylinderform abweichenden Abschnitt des Nippels in Richtung der Längsachse mindestens teilweise überdeckt. Vorzugsweise überdeckt eine von dem Klemmmittel des Klemmelements ausgebildete Klemmfläche den von der Zylinderform abweichenden Abschnitt des Nippels in Richtung der Längsachse mindestens teilweise. Dadurch ist gewährleistet, dass die Leitung dicht an dem von der Zylinderform abweichenden Abschnitt des Nippels anliegt. Es ist auch möglich, dass das Klemmmittel, insbesondere die von dem Klemmmittel ausgebildete Klemmfläche, den von der Zylinder-form abweichenden Abschnitt des Nippels vollständig überdeckt.

In einer Ausführungsart weist das Klemmmittel des Klemmelements einen die Leitung fixierenden Absatz auf, wobei eine den Absatz bildende Fläche mit der Längsachse des Grundkörper einen Winkel zwischen 20° und 80° einschließt, insbesondere zwischen 40° und 75° und vorzugsweise zwischen 45° und 60°. Durch eine solche verhältnismäßig steile Flanke wird die Leitung im geklemmten Zustand sicher an der Vorrichtung gehalten, insbesondere gegenüber Kräften, die in Richtung von dem Grundkörper wegweisend auf die Leitung einwirken, beispielsweise gegenüber Zugkräften an der Leitung und/oder gegenüber von Kräften, die infolge eines Überdrucks in der Leitung auftreten.

In einer Ausführungsart weist der Nippel auf seiner Innenfläche einen von der Zylinderform abweichenden Abschnitt auf, der in Richtung der Längsachse vorzugsweise im Bereich des von der Zylinderform abweichenden Abschnitts auf der Außenseite des Nippels angeordnet ist. Beispielsweise weist der Nippel auf seiner Innenfläche eine sich in Richtung auf das der Leitung zugewandte Ende des Nippels verbreiternde Kegelform oder eine sonstige gekrümmte und insbesondere teilsphärische Form auf, durch welche sich das der Leitung zugewandte Ende des Nippels hinsichtlich seiner Wandstärke verjüngt. An seinem der Leitung zugewandten stirnseitigen Ende kann der Nippel eine gekrümmte, insbesondere teilsphärische, oder plane Endfläche aufweisen, an welche die Leitung im geklemmten Zustand in Anlage bringbar ist und dadurch die Totraumfreiheit weiter verbessert ist.

In einer Ausführungsart weist der Nippel auf seiner Außenfläche einen weiteren von der Zylinderform abweichenden Abschnitt auf, an den die Leitung beim Aufschieben in Anlage bringbar ist. Auch der weitere Abschnitt kann eine in Richtung auf das der Leitung zugewandte Ende des Nippels verjüngende Kegelform aufweisen. Beim Aufschieben bildet der weitere Abschnitt einen Anschlag für die Leitung, deren stirnseitiges Ende innenseitig in Anlage an den weiteren Abschnitt kommt. Im weiteren von dem der Leitung zugewandten Ende wegweisenden Verlauf des Nippels kann das Klemmelement angeordnet sein, wobei zwischen dem weiteren Abschnitt, der den Anschlag für die Leitung bildet, und dem Klemmelement ein Freiraum vorgesehen sein kann, in den hinein sich die Leitung beispielsweise im Falle einer Erwärmung, infolge der Pressung und/oder als Toleranzausgleich ausdehnen kann, ohne die Dichtwirkung der Verbindung negativ zu beeinträchtigen.

In einer Ausführungsart weist der Grundkörper einen Aufnahmeraum für das Klemmelement auf, der radial innen vorzugsweise durch einen Abschnitt des Nippels begrenzt ist. Bei dem Zusammenbau der Vorrichtung kann das Klemmelement in diesen Aufnahmeraum eingesetzt werden, wobei der Aufnahmeraum jedenfalls radial innenseitig eine zylindrische Begrenzungswand aufweist, so dass auch eine automatische Zentrierung des Klemmelements in Bezug auf den Grundkörper gewährleistet ist.

In einer Ausführungsart ist der Aufnahmeraum radial außen durch ein sich in Richtung der Längsachse erstreckendes Begrenzungselement begrenzt, vorzugsweise durch einen um die Längsachse umlaufenden ringförmigen Steg. Das Begrenzungselement kann auch dafür verwendet werden, um das Klemmelement an dem Grundkörper festzulegen. Hierzu kann beispielsweise von radial außen eine Verformung des Begrenzungselements vorgenommen werden, durch die jedenfalls eine axiale Fixierung, vorzugsweise auch eine drehfeste Verbindung des Klemmelements mit dem Grundkörper, herstellbar ist. Beispielsweise kann das Begrenzungselement an seinem der Leitung zugewandten freien axialen Ende zur drehfesten Verbindung des Klemmelements mit dem Grundkörper abgebogen sein, insbesondere umbördelt sein. Alternativ oder ergänzend kann das Klemmelement auch mit dem Grundkörper durch Presspassung verbunden sein, beispielsweise auch mittels einer Rändelung an dem Klemmelement und/oder dem Grundkörper.

In einer Ausführungsart weist das Klemmelement vorzugsweise einstückig mehrere sich in Richtung der Längsachse erstreckende und radial federnd auslenkbare Klemmzungen auf. Die Klemmzungen können dabei die vorstehend beschriebenen Klemmmittel aufweisen, insbesondere die Klemmflächen ausbilden. Die Klemmzungen können über einen Verbindungsabschnitt, der insbesondere ringförmig ausgebildet sein kann, miteinander verbunden sein. Vorzugsweise sind die Klemmzungen und der Verbindungsabschnitt einstückig ausgebildet. Mittels des Verbindungsabschnitts kann das Klemmelement an den Grundkörper festgelegt sein.

In einer Ausführungsart weisen die Klemmzungen auf ihrer Außenseite einen von der Zylinderform abweichenden Abschnitt auf, insbesondere einen sich in Richtung auf das der Leitung zugewandte Ende verjüngenden kegelförmigen oder gekrümmt-geformten Abschnitt, der mit einer Anlagefläche eines Betätigungselements der Vorrichtung derart zusammenwirkt, dass beim Betätigen des Betätigungselements die Klemmzungen radial in Richtung auf die Leitung auslenkbar sind und dabei in klemmende Anlage an die Leitung bringbar sind. Beispielsweise können die Klemmzungen einen in Bezug auf die Längsachse kegelförmigen Abschnitt aufweisen, der mit einem entsprechenden kegelförmigen Abschnitt des Betätigungselements wie vorstehend beschrieben zusammenwirkt.

In einer Ausführungsart weist die Vorrichtung eine definierte Endstellung für das Klemmelement beim Klemmen der Leitung auf, die auch im Falle einer weiteren Betätigung eines Betätigungselements der Vorrichtung beibehalten wird, so dass eine Überlastung des Klemmelements oder der Leitung verhindert ist. Hierzu können die Klemmzungen auf ihrer Außenfläche einen weiteren Abschnitt aufweisen, der in dem die Leitung klemmenden ausgelenkten Zustand der Klemmzungen im Wesentlichen zylindrisch zur Längsachse des Grundkörpers ausgerichtet ist, und der mit dem Betätigungselement derart zusammenwirkt, dass bei einem weiteren Betätigen des Betätigungselements die Klemmzungen radial nicht weiter in Richtung auf die Leitung auslenkbar sind. Dadurch werden die Klemmzungen und mithin das Klemmmittel nur in einem vorgebbaren Umfang in Richtung auf die Leitung ausgelenkt, so dass eine definierte Endposition der Klemmzungen in Bezug auf die Leitung gegeben ist. Es kann daher nicht zu einer Überbeanspruchung der Leitung kommen, die für eine dauerhafte Dichtwirkung nachteilig sein könnte.

In einer Ausführungsart weist die Vorrichtung ein Betätigungselement für das Fixieren und/oder Lösen der Leitung auf, wobei das Betätigungselement unverlierbar an dem Grundkörper festlegbar ist. Das Betätigungselement kann gegenüber dem Grundkörper drehbar und/oder verschiebbar sein. In einer Ausführungsart weist das Betätigungselement im Wesentlichen eine Hülsenform auf und trägt ein Innengewinde, mittels dem das Betätigungselement auf dem ein korrespondierendes Außengewinde tragenden Grundkörper aufschraubbar ist. Das Außengewinde kann auch mehrgängig sein, insbesondere zweigängig, so dass gegebenenfalls auch mit weniger als zwei, insbesondere mit weniger als einer Umdrehung des Betätigungselements das Klemmelement betätigbar ist, beispielsweise in klemmende Anlage an die Leitung bringbar ist.

In einer Ausführungsart weist das Betätigungselement ein federnd auslenkbare Rastmittel auf, die beim erstmaligen Zusammenführen des Betätigungselements mit dem Grundkörper über ein von dem Grundkörper ausgebildetes korrespondierendes Rastmittel bewegbar ist und dadurch unverlierbar an dem Grundkörper festlegbar ist. Das Rastmittel des Betätigungselements kann beispielsweise durch einen radial nach innen vorstehenden Vorsprung an oder nahe dem vorzugsweise von der Leitung abgewandten Ende des Betätigungselements gebildet sein.

In einer Ausführungsart bildet der Grundkörper einen Anschlag für das Betätigen des Betätigungselements, insbesondere einen Anschlag für die Relativbewegung des Betätigungselements gegenüber dem Grundkörper, wobei das Betätigungselement in dem die Leitung fixierenden Zustand des Klemmelements in Anlage an dem Anschlag sein kann. Dadurch ist der die Leitung fixierende Zustand der Vorrichtung visuell erkennbar, beispielsweise weil ein zuvor vorhandener Spalt zwischen dem Betätigungselement und dem Grundkörper verschwindet. Der Anschlag kann durch einen stufenförmigen Absatz des Grundkörpers gebildet sein. Das Betätigungselement kann in dem die Leitung fixierenden Zustand bündig in Anlage an dem Absatz des Grundkörpers sein, so dass dieser Zustand auch taktil erkennbar ist.

Weitere Vorteile, Merkmale und Einzelheiten der Erfindung ergeben sich aus den Unteransprüchen sowie der nachfolgenden Beschreibung, in der unter Bezugnahme auf die Zeichnung ein Ausführungsbeispiel im Einzelnen beschrieben ist. Dabei können die in den Ansprüchen und in der Beschreibung erwähnten Merkmale jeweils einzeln für sich oder in beliebiger Kombination erfindungswesentlich sein.

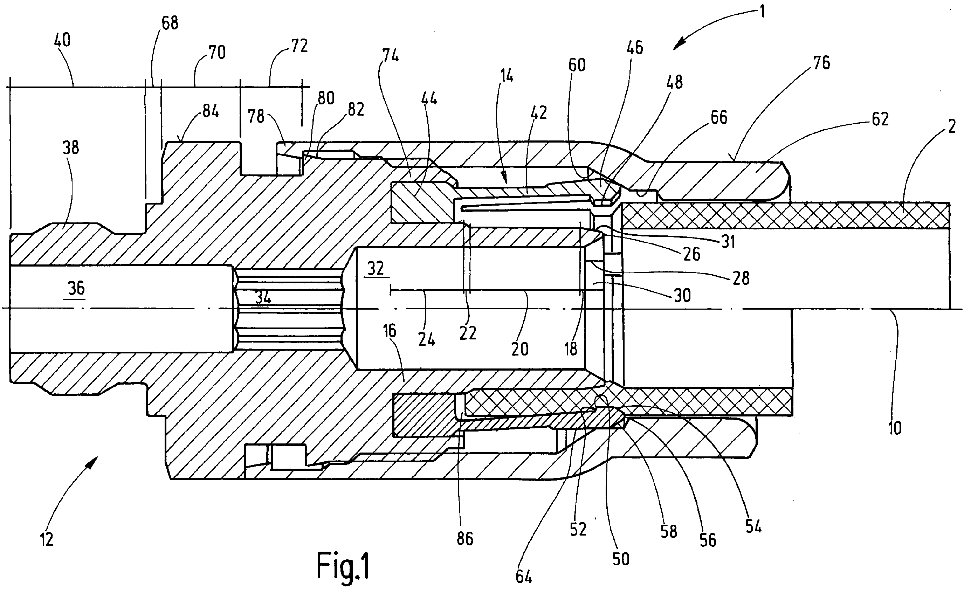

- Fig. 1: zeigt einen Schnitt durch ein Ausführungsbeispiel einer erfindungsgemäßen Vorrichtung.

Die Fig. 1 zeigt einen Schnitt durch ein Ausführungsbeispiel einer erfindungsgemäßen Vorrichtung 1 zum Verbinden mit einem Ende einer röhrenartigen Leitung 2, wobei in der oberen Bildhälfte der Fig. 1 der Zustand dargestellt ist, in dem die Leitung 2 noch nicht mit der Vorrichtung 1 verbunden ist, und in der unteren Bildhälfte der Fig. 1 der Zustand dargestellt ist, in dem die Leitung 2 mit der Vorrichtung 1 verbunden und an der Vorrichtung 1 fixiert ist.

Die Vorrichtung 1 weist einen eine Längsachse 10 aufweisenden Grundkörper 12 auf und ein Klemmelement 14, mittels dem die Leitung 2 an der Vorrichtung 1 lösbar fixierbar ist, und das im Ausführungsbeispiel als sogenannte Spannzange ausgebildet ist. Der Grundkörper 12 weist in einem der Leitung 2 zugewandten Bereich, insbesondere auf seiner der Leitung 2 zugewandten Seite, einen Nippel 16 auf, auf den die Leitung 2 aufschiebbar ist. Der Nippel 16 weist auf seiner Außenfläche einen von der Zylinderform abweichenden und radial außen eine Kegelform 31 aufweisenden Abschnitt 18 auf, an der die Leitung 2 mittels des Klemmelements 14 in dichtender Anlage fixierbar ist, wie sich insbesondere aus der unteren Bildhälfte der Fig. 1 ergibt. Dieser Abschnitt 18 ist im Ausführungsbeispiel endseitig an dem Nippel 16 angeordnet.

In Richtung von der Leitung 2 wegweisend schließt sich an diesen Abschnitt 18 auf der Außenseite des Nippels 16 ein zylindrischer Abschnitt 20 an, auf den die Leitung 2 aufschiebbar ist, wobei der Außendurchmesser des zylindrischen Abschnitts 20 mit dem Innendurchmesser der Leitung 2 im Wesentlichen übereinstimmt, insbesondere hiervon weniger als 10 %, insbesondere weniger als 5 % und vorzugsweise weniger als 2 % abweicht, insbesondere geringer ist. Im Ausführungsbeispiel schließt unmittelbar an den zylindrischen Abschnitt 20 ein weiterer von der Zylinderform abweichender Abschnitt 22 an, der sich in Richtung auf das der Leitung 2 zugewandte Ende des Nippels 16 verjüngt und kegelförmig ausgebildet ist. Bis zu diesem weiteren Abschnitt 22 ist die Leitung 2 mit geringer Kraft aufschiebbar. An den weiteren Abschnitt 22 schließt sich ein weiterer zylindrischer Abschnitt 24 an, der radial innenseitig einen Aufnahmeraum für das Klemmelement 14 begrenzt.

Der von der Zylinderform abweichende Abschnitt 18 des Nippels 16 weist eine sich in Richtung auf das der Leitung 2 zugewandte Ende des Nippels 16 verjüngende Kegelform 31 auf, wobei der Kegelwinkel mehr als 3° und weniger als 30° beträgt, vorzugsweise mehr als 5° und weniger als 20°, und im Ausführungsbeispiel zwischen 8° und 15° beträgt. Der Abschnitt 18 wird von dem radial außenseitigen Endabschnitt des Nippels 16 gebildet. Daran schließt sich unter Ausbildung einer Kante oder mittels einer Verrundung eine plane oder gekrümmte, insbesondere teilsphärische stirnseitige Endfläche 26 des Nippels 16 an, die nach radial innen in einen von der Zylinderform abweichenden Abschnitt 28 übergeht, der radial innen eine Kegelfläche 30 bildet, die sich zum freien Ende hin aufweitet. Unter Ausbildung einer Kante oder mittels einer Verrundung schließt sich daran eine im Wesentlichen zylindrische Öffnung 32 an, die der Leitungsfortführung dient, und die sich axial bis über die Länge des Nippels 16 hinaus erstreckt, um anschließend im Grundkörper 12 über eine Verbindungsöffnung 34, die ebenfalls vorzugsweise zylindrisch ist, insbesondere im Querschnitt polygonal ist, vorzugsweise einen Sechskant bildet, in eine zylindrische Öffnung 36 überzugehen, die bis zu dem der Leitung 2 gegenüberliegenden axialen Ende des Grundkörpers 12 reicht, insbesondere bis in einen Bereich eines auf der Außenseite ein Außengewinde 38 tragenden Anschlussstutzens 40.

Das Klemmelement weist mehrere sich in Richtung der Längsachse 10 erstreckende und radial zur Längsachse 10 federnd auslenkbare Klemmzungen 42 auf, die an ihrem der Leitung 2 abgewandten Ende über einen Verbindungsring 44 miteinander verbunden sind, und die auch als Segmente einer Spannzange ausgebildet sein können. Das dem freien Ende des Nippels 16 zugewandte Ende des Verbindungsrings 44 ist von dem weiteren von der Zylinderform abweichenden Abschnitt 22 des Nippels 16 in Richtung der Längsachse (10) beabstandet, so dass dadurch ein Freiraum 86 gebildet ist, in den hinein sich die Leitung 2 beispielsweise aufgrund von Erwärmung bedingter Ausdehnung erstrecken kann. An ihrem der Leitung 2 und mithin dem freien Ende des Nippels 16 zugewandten Ende weisen die Klemmzungen 42 ein Klemmmittel 46 auf, das radial innenseitig eine gekrümmte, insbesondere teilsphärische bzw. teilkugelförmige, oder plane Klemmfläche 48 ausbildet, die beim Auslenken der Klemmzunge 42 in klemmende Anlage an die Außenumfangsfläche 2 der Leitung kommt. In dem in der oberen Bildhälfte der Fig. 1 dargestellten Ausgangszustand zum Einführen der Leitung 2 ist die Klemmzunge 42 so weit radial nach außen ausgelenkt, dass die Klemmfläche 48 im Wesentlichen mit dem Außendurchmesser der einzuführenden Leitung 2 fluchtet, so dass beim Einführen der Leitung 2 keine Pressung auf die Leitung 2 erfolgt, sondern erst wenn zum Fixieren der Leitung 2 die Klemmzunge 42 ausgelenkt wird und dabei der in der unteren Bildhälfte der Fig. 1 dargestellte Endzustand eingestellt wird..

An die Klemmfläche 48 schließt sich in Richtung auf den Verbindungsring 44 eine Klemmkante an, die durch einen Absatz 50 gebildet ist, mittels dem die Leitung 2 im geklemmten Zustand an der Vorrichtung 1, insbesondere an dem Grundkörper 12, auch gegen Zugkräfte auf die Leitung 2 gehalten ist. Eine den Absatz 50 bildende Fläche 52 schließt in dem in der unteren Bildhälfte der Fig. 1 dargestellten die Leitung 2 klemmenden Zustand mit der Längsachse 10 einen Winkel zwischen 20° und 80° ein, insbesondere zwischen 40° und 75° und vorzugsweise zwischen 45° und 60°.

In Richtung auf das der Leitung 2 zugewandte freie Ende geht die Klemmfläche 48 des Klemmmittels 46 radial innen über eine sich aufweitende kegelförmige Fläche 54, die auch eine Einführschräge oder Zentrierhilfe für die in die Vorrichtung 1 einzuführende Leitung 2 bilden kann, in eine plane stirnseitige Endfläche 56 über, die eine Erstreckung in Radialrichtung von weniger als 50 %, vorzugsweise weniger als 30 %, der radialen Erstreckung des Klemmmittels 46 im Bereich der Klemmfläche 48 aufweist. Daran schließt sich radial außen ein von der Zylinderform abweichender Abschnitt 58 an, im Ausführungsbeispiel ein sich in Richtung auf das der Leitung 2 zugewandte Ende verjüngender kegelförmiger Abschnitt 58. Dieser wirkt mit einer Anlagefläche 60 eines Betätigungselements 62 der Vorrichtung 1 derart zusammen, dass beim Betätigen des Betätigungselements 62 die Klemmzungen 42 radial in Richtung auf die Leitung 2 auslenkbar sind und in klemmende Anlage an die Leitung 2 bringbar sind. An den kegelförmigen Abschnitt 58 schließt sich auf der Außenfläche des Klemmmittels 48 ein bis in den Bereich der Klemmzunge 42 hineinragender weiterer Abschnitt 64 an, der in dem die Leitung 2 klemmenden ausgelenkten Zustand der Klemmzungen 42 zylindrisch zur Längsachse 10 des Grundkörpers 12 ausgerichtet ist und mit einer weiteren Anlagefläche 66 des Betätigungselements 62 derart zusammenwirkt, dass bei einem weiteren Betätigen des Betätigungselements 62 die Klemmzungen 42 radial nicht weiter in Richtung auf die Leitung 2 auslenkbar sind. Die weitere Anlagefläche 66 des Betätigungselements 62 ist zylindrisch, insbesondere kreiszylindrisch, parallel zur Längsachse 10.

Wie in der unteren Bildhälfte der Fig. 1 dargestellt, überdeckt das Klemmmittel 46, insbesondere die Klemmfläche 48 den von der Zylinderform abweichenden Abschnitt 18 des Nippels 16 über das axiale Ende des Nippels 16 hinaus, so dass die Leitung 2 nicht nur in dichte Anlage an den Abschnitt 18 gehalten ist, sondern sich auch an die stirnseitige Endfläche 26 anlegt, jedenfalls sich auch an einen Übergang von dem von der Zylinderform abweichenden Abschnitt 18, insbesondere der Kegelfläche 31, zu dem stirnseitigen Ende des Nippels 16 anlegt, und dadurch eine totraumfreie Verbindung zwischen der Leitung 2 und dem Nippel 16 und damit der Vorrichtung 1 bereitstellt. Die Klemmfläche 48 endet in der anderen Richtung im Bereich des Abschnitts 18, so dass der die Leitung 2 haltende Absatz 50 des Klemmmittels im Bereich des Abschnitts 18 angeordnet ist. Die Flächenpressung auf die Leitung 2 ist durch die Wahl der Größe der Klemmfläche 48 derart begrenzt, dass ein Fließen des Werkstoffes der Leitung 2 so weit reduziert ist, dass die Dichtwirkung dadurch nicht nachteilig beeinträchtigt ist.

Ausgehend von dem der Leitung 2 abgewandten Ende schließt sich an den Anschlussstutzen 40 des Grundkörpers 12 ein zylindrischer Abschnitt 68 an, insbesondere ein kreiszylindrischer Abschnitt. In Richtung auf die Leitung 2 schließt sich daran unter Vergrößerung der radialen Erstreckung ein gegebenenfalls ebenfalls zylindrischer Abschnitt 70 an, der eine Werkzeugangriffsfläche 84 aufweist und beispielsweise durch einen Außensechskant gebildet ist. Daran schließt sich ein weiterer Abschnitt 72 an, der eine Ringnut bildet, in die ein Endabschnitt des Betätigungselements 62 eingreifen kann. Unter Ausbildung eines Absatzes geht der weitere Abschnitt 72 in einen im Wesentlichen zylindrischen, insbesondere kreiszylindrischen, und ein Außengewinde aufweisenden Abschnitt über, der auch ein Begrenzungselement für den das Klemmelement 14 aufnehmenden Aufnahmeraum des Grundkörpers 12 bildet, insbesondere einen um die Längsachse 10 umlaufenden ringförmigen Steg 74. An seinem freien axialen Ende ist dieser Steg, wie in der Fig. 1 dargestellt, umgebogen und bildet dadurch eine drehfeste Verbindung zwischen Grundkörper 12 und Klemmelement 14. Das Klemmelement 14 ist vorzugsweise auch in Richtung der Längsachse 10 unverschiebbar an dem Grundkörper 12 festgelegt.

Das Betätigungselement 62 ist im Wesentlichen hülsenförmig ausgebildet und weist an seinem der Leitung 2 zugewandten Ende einen Abschnitt mit einem reduzierten Außendurchmesser auf, der außenseitig eine Werkzeugangriffsfläche 76 ausbildet, beispielsweise einen Außensechskant. An seinem von der Leitung 2 abgewandten Ende weist das Betätigungselement 62 ein federnd auslenkbares Rastmittel 78 auf, das im Ausführungsbeispiel durch einen nach radial innen vorstehenden hakenförmigen Vorsprung gebildet ist. Beim erstmaligen Zusammenführen des Betätigungselements 62, insbesondere beim erstmaligen Aufschrauben des ein Innengewinde aufweisenden Betätigungselements 62 auf den Grundkörper 12, wird das Rastmittel 78 über ein von dem Grundkörper 12 ausgebildetes korrespondierendes Rastmittel 80 bewegt und dadurch unverlierbar an dem Grundkörper 12 festgelegt. Das korrespondierende Rastmittel 80 ist im Ausführungsbeispiel durch einen Absatz am Übergang zu dem die Nut bildenden Abschnitt 72 gebildet, wobei eine diesen Absatz bildende Fläche 82 kegelförmig ist und mit der Längsachse 10 einen Winkel von weniger als 30°, vorzugsweise weniger als 20° und insbesondere etwa 10° einschließt.

Am Übergang von dem die Nut bildenden Abschnitt 72 zu dem die Werkzeugangriffsfläche 84 bildenden Abschnitt 70 bildet der Grundkörper 12 einen Anschlag für das Betätigen des Betätigungselements 62, wobei das Betätigungselement 62 in dem die Leitung 2 fixierenden Zustand des Klemmelements 14, wie in der unteren Bildhälfte der Fig. 1 dargestellt, in Anlage an dem Anschlag ist, insbesondere bündig in Anlage an dem Anschlag ist, und daher ein zuvor vorhandener Spalt zwischen dem Absatz und dem Betätigungselement 62 geschlossen ist. Dadurch ist der klemmende Zustand visuell und/oder taktil erfassbar.

## Patentansprüche

1. Vorrichtung (1) zum Verbinden mit einem Ende einer röhrenartigen Leitung (2), insbesondere Schlauch-Schnellverbinder, mit einem eine Längsachse (10) aufweisenden Grundkörper (12) und mit einem Klemmelement (14), mittels dem die Leitung (2) an der Vorrichtung (1) lösbar fixierbar ist, wobei der Grundkörper (12) einen Nippel (16) aufweist, auf den die Leitung (2) aufschiebbar ist, wobei der Nippel (16) auf seiner Außenfläche einen von der Zylinderform abweichenden Abschnitt (18) aufweist, an dem die Leitung (2) mittels des Klemmelements (14) in dichtender Anlage fixierbar ist, wobei das Klemmelement (14) ein Klemmmittel (46) aufweist, **dadurch gekennzeichnet, dass** das Klemmmittel (46) in Richtung der Längsachse (10) über den von der Zylinderform abweichenden Abschnitt (18) des Nippels (16) und in Richtung der Längsachse (10) über das der Leitung (2) zugewandte Ende des Nippels (16) hinausragt.

2. Vorrichtung (1) nach Anspruch 1, **dadurch gekennzeichnet, dass** der von der Zylinderform abweichende Abschnitt (18) eine sich in Richtung auf das der Leitung (2) zugewandte Ende des Nippels (16) verjüngende Kegelform (31) aufweist.

3. Vorrichtung (1) nach Anspruch 1 oder 2, **dadurch gekennzeichnet, dass** der Nippel (16) den von der Zylinderform abweichenden Abschnitt (18) an oder nahe seinem der Leitung (2) zugewandten Ende aufweist.

4. Vorrichtung (1) nach einem der vorgenannten Ansprüche, **dadurch gekennzeichnet, dass** das Klemmelement (14) für den klemmenden Kontakt mit der Leitung (2) das Klemmmittel (46) aufweist, das im Bereich des von der Zylinderform abweichenden Abschnitts (18) des Nippels (16) in Anlage an die Leitung (2) bringbar ist.

5. Vorrichtung (1) nach einem der vorgenannten Ansprüche, **dadurch gekennzeichnet, dass** das Klemmmittel (46) eine Klemmfläche (48) aufweist, und dass die Klemmfläche (48) ausreichend groß ist, um beim Klemmen die Flächenpressung auf die Leitung (2) auf einen vorgebbaren Wert zu begrenzen.

6. Vorrichtung (1) nach einem der vorgenannten Ansprüche, **dadurch gekennzeichnet, dass** das Klemmmittel (46) den von der Zylinderform abweichenden Abschnitt (18) des Nippels (16) in Richtung der Längsachse (10) mindestens teilweise überdeckt, insbesondere dass eine von dem Klemmmittel des Klemmelements (14) ausgebildete Klemmfläche (48) den von der Zylinderform abweichenden Abschnitt (18) des Nippels (16) in Richtung der Längsachse (10) mindestens teilweise überdeckt.

7. Vorrichtung (1) nach einem der vorgenannten Ansprüche, **dadurch gekennzeichnet, dass** das Klemmmittel (46) einen die Leitung (2) fixierenden Absatz (50) aufweist, und dass eine den Absatz (50) bildende Fläche (52) mit der Längsachse (10) einen Winkel von mehr als 30°, insbesondere mehr als 45° und vorzugsweise mehr als 55° einschließt.

8. Vorrichtung (1) nach einem der vorgenannten Ansprüche, **dadurch gekennzeichnet, dass** der Nippel (16) auf seiner Innenfläche einen von der Zylinderform abweichenden Abschnitt (28) aufweist, der in Richtung der Längsachse (10) vorzugsweise im Bereich des von der Zylinderform abweichenden Abschnitts (18) auf der Außenseite des Nippels (16) angeordnet ist.

9. Vorrichtung (1) nach einem der vorgenannten Ansprüche, **dadurch gekennzeichnet, dass** der Nippel (16) auf seiner Außenfläche einen weiteren von der Zylinderform abweichenden Abschnitt (22) aufweist, an den die Leitung (2) beim Aufschieben in Anlage bringbar ist.

10. Vorrichtung (1) nach einem der vorgenannten Ansprüche, **dadurch gekennzeichnet, dass** der Grundkörper (12) einen Aufnahmeraum für das Klemmelement (14) aufweist, und dass der Aufnahmeraum radial innen vorzugsweise durch einen Abschnitt des Nippels (16) begrenzt ist.

11. Vorrichtung (1) nach einem der vorgenannten Ansprüche, **dadurch gekennzeichnet, dass** der Grundkörper (12) einen Aufnahmeraum für das Klemmelement (14) aufweist, und dass der Aufnahmeraum radial außen durch ein sich in Richtung der Längsachse (10) erstreckendes Begrenzungselement begrenzt ist, vorzugsweise durch einen um die Längsachse (10) umlaufenden ringförmigen Steg (74) begrenzt ist.

12. Vorrichtung (1) nach einem der vorgenannten Ansprüche, **dadurch gekennzeichnet, dass** das Klemmelement (14) vorzugsweise einstückig mehrere sich in Richtung der Längsachse (10) erstreckende und radial federnd auslenkbare Klemmzungen (42) aufweist.

13. Vorrichtung (1) nach Anspruch 12,
**dadurch gekennzeichnet, dass** die
Klemmzungen (42) auf ihrer Außenfläche einen von der Zylinderform abweichenden Abschnitt aufweisen, insbesondere einen sich in Richtung auf das der Leitung (2) zugewandte Ende verjüngenden kegelförmigen Abschnitt (58), der mit einer Anlagefläche (60) eines Betätigungselements (62) derart zusammenwirkt, dass beim Betätigen des Betätigungselements (62) die Klemmzungen (42) radial in Richtung auf die Leitung (2) auslenkbar sind und in klemmende Anlage an die Leitung (2) bringbar sind.

14. Vorrichtung (1) nach Anspruch 12 oder 13,
**dadurch gekennzeichnet,**
**dass** die Klemmzungen (42) auf ihrer Außenfläche einen weiteren Abschnitt (64) aufweisen, der in dem die Leitung (2) klemmenden ausgelenkten Zustand der Klemmzungen (42) zylindrisch zur Längsachse (10) des Grundkörpers (12) ausgerichtet ist und mit einer weiteren Anlagefläche (66) eines Betätigungselements (62) derart zusammenwirkt, dass beim weiteren Betätigen des Betätigungselements (62) die Klemmzungen (42) radial nicht weiter in Richtung auf die Leitung (2) auslenkbar sind.

15. Vorrichtung (1) nach einem der vorgenannten Ansprüche, **dadurch gekennzeichnet, dass** die Vorrichtung (1) ein Betätigungselement (62) für das Fixieren und/oder Lösen der Leitung (2) aufweist, und dass das Betätigungselement (62) unverlierbar an dem Grundkörper (12) festgelegt ist.

16. Vorrichtung (1) nach einem der vorgenannten Ansprüche, **dadurch gekennzeichnet, dass** das Betätigungselement (62) ein federnd auslenkbares Rastmittel (78) aufweist, das beim erstmaligen Zusammenführen des Betätigungselements (62) mit dem Grundkörper (12) über ein von dem Grundkörper (12) ausgebildetes korrespondierendes Rastmittel (80) bewegbar ist und dadurch unverlierbar an dem Grundkörper (12) festlegbar ist.

17. Vorrichtung (1) nach einem der vorgenannten Ansprüche, **dadurch gekennzeichnet, dass** der Grundkörper (12) einen Anschlag für das Betätigen des Betätigungselements (62) bildet, und dass das Betätigungselement (62) in dem die Leitung (2) fixierenden Zustand des Klemmelements (14) in Anlage an dem Anschlag ist.

## Claims

1. A device (1) for connecting to an end of a tubular conduit (2), in particular a quick hose connector, comprising a base body (12) that has a longitudinal axis (10) and comprising a clamping element (14) by means of which the conduit (2) can be fixed detachably to the device (1), the base body (12) having a nipple (16) onto which the conduit (2) can be pushed, the nipple (16) having on its outer surface a section (18) deviating from the cylindrical shape and to which the conduit (2) can be fixed with sealing contact by means of the clamping element (14), the clamping element (14) having a clamping means (46), **characterised in that** the clamping means (46) projects in the direction of the longitudinal axis (10) over the section (18) of the nipple (16) deviating from the cylindrical shape and in the direction of the longitudinal axis (10) beyond the end of the nipple (16) facing the conduit (2).

2. The device (1) according to Claim 1, **characterised in that** the section (18) deviating from the cylindrical shape has a cone shape (31) that tapers towards the end of the nipple (16) facing the conduit (2).

3. The device (1) according to Claim 1 or 2, **characterised in that** the nipple (16) has the section (18) deviating from the cylindrical shape on or near to its end facing the conduit (2).

4. The device (1) according to any of the claims specified above, **characterised in that** for the clamping contact with the conduit (2) the clamping element (14) has the clamping means (46) that can be brought into contact with the conduit (2) in the region of the section (18) of the nipple (16) deviating from the cylindrical shape.

5. The device (1) according to any of the claims specified above, **characterised in that** the clamping means (46) has a clamping surface (48) and that the clamping surface (48) is sufficiently large in order, upon clamping, to limit the surface pressure acting on the conduit (2) to a pre-definable value.

6. The device (1) according to any of the claims specified above, **characterised in that** the clamping means (46) at least partially covers the section (18) of the nipple (16) deviating from the cylindrical shape in the direction of the longitudinal axis (10), in particular that a clamping surface (48) formed by the clamping means of the clamping element (14) at least partially covers the section (18) of the nipple (16) deviating from the cylindrical shape in the direction of the longitudinal axis (10).

7. The device (1) according to any of the claims specified above, **characterised in that** the clamping means (46) has a shoulder (50) that fixes the conduit (2), and that a surface (52) forming the shoulder (50) encloses an angle of more than 30°, in particular more than 45°, and preferably more than 55°, with the longitudinal axis (10).

8. The device (1) according to any of the claims specified above, **characterised in that** the nipple (16) has on its inner surface a section (28) deviating from the cylindrical shape which is disposed in the direction of the longitudinal axis (10), preferably in the region of the section (18) deviating from the cylindrical shape, on the outside of the nipple (16).

9. The device (1) according to any of the claims specified above, **characterised in that** the nipple (16) has on its outer surface an additional section (22) deviating from the cylindrical shape with which the conduit (2) can be brought into contact when pushed on.

10. The device (1) according to any of the claims specified above, **characterised in that** the base body (12) has a receiving space for the clamping element (14), and that the receiving space is preferably delimited radially to the inside by a section of the nipple (16).

11. The device (1) according to any of the claims specified above, **characterised in that** the base body (12) has a receiving space for the clamping element (14), and that the receiving space is delimited radially to the outside by a limitation element extending in the direction of the longitudinal axis (10), preferably by an annular bar (74) running round the longitudinal axis (10).

12. The device (1) according to any of the claims specified above, **characterised in that** the clamping element (14) preferably has, integrally, a number of clamping tongues (42) extending in the direction of the longitudinal axis (10) and that can be elastically deflected radially.

13. The device (1) according to Claim 12, **characterised in that** the clamping tongues (42) have on their outer surface a section deviating from the cylindrical shape, in particular a conical section (58) that tapers towards the end facing the conduit (2) and that co-operates with a contact surface (60) of an actuation element (62) such that upon actuating the actuation element (62) the clamping tongues (42) can be deflected radially towards the conduit (2) and can thus be brought into clamping contact with the conduit (2).

14. The device (1) according to Claim 12 or 13, **characterised in that** the clamping tongues (42) have on their outer surface an additional section (64) that, in the deflected state of the clamping tongues (42) clamping the conduit (2), is aligned cylindrically to the longitudinal axis (10) of the base body (12) and co-operates with an additional contact surface (66) of an actuation element (62) such that upon further actuation of the actuation element (62) the clamping tongues (42) can not be deflected any further radially towards the conduit (2).

15. The device (1) according to any of the claims specified above, **characterised in that** the device (1) has an actuation element (62) for fixing and/or releasing the conduit (2), and that the actuation element (62) can be fixed undetachably to the base body (12).

16. The device (1) according to any of the claims specified above, **characterised in that** the actuation element (62) has a latching means (62) that can be deflected elastically and which, upon bringing together the actuation element (62) and the base body (12) for the first time can be moved over a corresponding latching means (80) formed by the base body (12) and can in this way be fixed undetachably to the base body (12).

17. The device (1) according to any of the claims specified above, **characterised in that** the base body (12) forms a stop for the actuation of the actuation element (62), and that, in the state of the clamping element (14) fixing the conduit (2), the actuation element (62) is in contact with the stop.

## Revendications

1. Dispositif (1) de connexion à une extrémité d'un conduit (2) tubulaire, notamment d'un raccord rapide souple, comprenant un corps (2) de base, ayant un axe (10) longitudinal et comprenant un élément (14) de serrage, au moyen duquel le conduit (2) peut être immobilisé de manière amovible sur le dispositif (1), dans lequel le corps (12) de base a un embout (16) sur lequel le conduit (2) peut coulisser, l'embout (16) ayant, sur sa surface extérieure, un tronçon (18), qui s'écarte de la forme cylindrique et sur lequel le conduit (2) peut être immobilisé en application avec étanchéité au moyen de l'élément (14) de serrage, dans lequel l'élément (14) de serrage a un moyen (46) de serrage, **caractérisé en ce que** le moyen (46) de serrage dépasse, dans la direction de l'axe (10) longitudinal, du tronçon (18) de l'embout (16) s'écartant de la forme cylindrique et dans la direction de l'axe (10) longitudinal, de l'extrémité de l'embout (16) tournée vers le conduit (2).

2. Dispositif (1) suivant la revendication 1, **caractérisé en ce que** le tronçon (18) s'écartant de la forme cylindrique a une forme (31) conique se rétrécissant dans la direction allant vers l'extrémité de l'embout (16) tournée vers le conduit (2).

3. Dispositif (1) suivant la revendication 1 ou 2, **caractérisé en ce que** l'embout (16) a un tronçon (18) s'écartant de la forme cylindrique à l'extrémité tournée vers le conduit (2) ou près de cette extrémité.

4. Dispositif (1) suivant l'une des revendications précédentes, **caractérisé en ce que** l'élément (14) de serrage a, pour le contact de serrage avec le conduit (2), le moyen (46) de serrage, qui peut, dans la région du tronçon (18) de l'embout (16) s'écartant de la forme cylindrique, être mis en application sur le conduit (2).

5. Dispositif (1) suivant l'une des revendications précédentes, **caractérisé en ce que** le moyen (46) de serrage a une surface (48) de serrage et **en ce que** la surface (48) de serrage est suffisamment grande pour, lors du serrage, délimiter la surface de pression sur le conduit (2) à une valeur pouvant être donnée à l'avance.

6. Dispositif (1) suivant l'une des revendications précédentes, **caractérisé en ce que** le moyen (46) de serrage recouvre, au moins en partie, dans la direction de l'axe (10) longitudinal, le tronçon (18) de l'embout (16) s'écartant de la forme cylindrique, notamment **en ce qu'**une surface (48) de serrage formée par le moyen de serrage de l'élément (14) de serrage recouvre, au moins en partie, dans la direction de l'axe (10) longitudinal, le tronçon (18) s'écartant de la forme cylindrique.

7. Dispositif (1) suivant l'une des revendications précédentes, **caractérisé en ce que** le moyen (46) de serrage a un ressaut (50) immobilisant le conduit (2) et **en ce qu'**une surface (52) formant le ressaut (50) fait, avec l'axe (10) longitudinal, un angle de plus de 30°, notamment de plus de 45° et, de préférence, de plus de 55°.

8. Dispositif (1) suivant l'une des revendications précédentes, **caractérisé en ce que** l'embout (16) a, sur sa surface intérieure, un tronçon (28), qui s'écarte de la forme cylindrique et qui, dans la région de l'axe (10) longitudinal, de préférence, dans la région du tronçon (18) s'écartant de la forme cylindrique, est monté sur le côté extérieur de l'embout (16).

9. Dispositif (1) suivant l'une des revendications précédentes, **caractérisé en ce que** l'embout (16) a, sur sa surface extérieure, un autre tronçon (22), qui s'écarte de la forme cylindrique et sur lequel le conduit (2) peut être mis en application lors du coulissement.

10. Dispositif (1) suivant l'une des revendications précédentes, **caractérisé en ce que** le corps (12) de base a un espace de réception de l'élément (14) de serrage et **en ce que** l'espace de réception est délimité radialement vers l'intérieur, de préférence, par un tronçon de l'embout (16).

11. Dispositif (1) suivant l'une des revendications précédentes, **caractérisé en ce que** le corps (12) de base a un espace de réception de l'élément (14) de serrage et **en ce que** l'espace de réception est délimité radialement vers l'extérieur par un élément de délimitation s'étendant dans la direction de l'axe (10) longitudinal, de préférence, par un talon (74) annulaire faisant le tour de l'axe (10) longitudinal.

12. Dispositif (1) suivant l'une des revendications précédentes, **caractérisé en ce que** l'élément (14) de serrage a, de préférence, d'une seule pièce, plusieurs languettes (42) de serrage s'étendant dans la direction de l'axe (10)
longitudinal et pouvant être déviés élastiquement radialement.

13. Dispositif (1) suivant la revendication 12, **caractérisé en ce que** les languettes (42) de serrage ont, sur leur surface extérieure, un tronçon s'écartant de la forme cylindrique, notamment un tronçon (58) conique se rétrécissant dans la direction allant vers l'extrémité tournée vers le conduit (2), tronçon qui coopère avec la surface (60) d'application d'un élément (62) d'actionnement, de manière à ce que, lorsque l'élément (62) d'actionnement est actionné, les languettes (42) de serrage peuvent être déviées radialement dans la direction allant vers le conduit (2) et peuvent être mises en application avec serrage sur le conduit (2).

14. Dispositif (1) suivant la revendication 12 ou 13, **caractérisé en ce que** les languettes (42) de serrage ont, sur leur surface extérieure, un autre tronçon (64), qui, dans l'état des languettes (42) déviées en serrant le conduit (2), est dirigé d'une manière cylindrique par rapport à l'axe (10) longitudinal du corps (12) de base et qui coopère avec une autre surface (66) d'application d'un élément (62) d'actionnement, de manière à ce que, lorsqu'on continue à actionner l'élément (62) d'actionnement, les languettes (42) de serrage ne peuvent pas continuer à être déviées radialement dans la direction allant vers le conduit (2).

15. Dispositif (1) suivant l'une des revendications précédentes, **caractérisé en ce que** le dispositif (1) a un élément (62) d'actionnement pour l'immobilisation et/ou la libération du conduit (2) et **en ce que** l'élément (62) d'actionnement est fixé de manière imperdable au corps (12) de base.

16. Dispositif (1) suivant l'une des revendications précédentes, **caractérisé en ce que** l'élément (62) d'actionnement a un moyen (78) d'encliquetage pouvant être dévié élastiquement, qui, lorsque l'élément (62) d'actionnement est réuni pour la première fois au corps (12) de base, peut être déplacé, par un moyen (80) d'encliquetage correspondant constitué par le corps (12) de base, et ainsi être fixé de manière imperdable au corps (12) de base.

17. Dispositif (1) suivant l'une des revendications précédentes, **caractérisé en ce que** le corps (12) de base forme une butée pour l'actionnement de l'élément (62) d'actionnement et **en ce que** l'élément (62) d'actionnement vient, dans l'état de l'élément (14) de serrage immobilisant le conduit (2), en application sur la butée.
